# EUROPÄISCHE PATENTANMELDUNG

(11) **EP 4 524 508 A1**
(43) Veröffentlichungstag der Anmeldung: **19.03.2025**
(21) Anmeldenummer: 24201169.0
(22) Anmeldetag: 18.09.2024
(51) Int. Cl.: G01B 7/06

(54) **VERFAHREN UND VORRICHTUNG ZUM BESTIMMEN VON EIGENSCHAFTEN VON AUS PAPIER ODER PAPPE HERGESTELLTEN BEHÄLTNISSEN**

(30) Priorität: 18.09.2023 DE 102023125199
(71) Anmelder: Krones AG, 93073 Neutraubling (DE)
(72) Erfinder: Niedermeier, Anton, 93073 Neutraubling (DE); Piana, Stefan, 93073 Neutraubling (DE)
(74) Vertreter: Bittner, Bernhard

(57) **Zusammenfassung**

Verfahren zum Bestimmen von Behältniseigenschaften, wobei ein zu untersuchendes Behältnis (10) entlang eines vorgegebenen Transportpfads transportiert wird und dieses Behältnis (10) einen Bodenbereich (10a), einen Grundkörper (10b) der zu Aufnahme eines Füllguts dient und einen Mündungsbereich aufweist und wobei dieses Behältnis (10) wenigstens abschnittsweise aus einem Papier- oder Pappe enthaltenden Material besteht, dadurch gekennzeichnet, dass wenigstens eine Wandung (12) des Behältnisses (10) zwischen zwei Elektroden (4, 6) angeordnet wird und an die Elektroden (4, 6) eine elektrische Spannung oder ein elektrisches Wechselfeld angelegt wird und wenigstens eine elektrische Größe bestimmt wird, welche für ein elektrisches Feld, welches zwischen Elektroden aufgebaut wird, charakteristisch ist und aus dieser elektrischen Größe wenigstens eine für das Behältnis (10) charakteristische Größe ermittelt wird.

## Beschreibung

Die vorliegende Erfindung bezieht sich auf ein Verfahren und eine Vorrichtung zum Bestimmen von Behältniseigenschaften. Derartige Verfahren und Vorrichtungen sind aus dem Stand der Technik seit langem bekannt. So ist es beispielsweise bekannt, dass bei der Herstellung von Kunststoffbehältnissen Eigenschaften derselben ermittelt werden, wie beispielsweise eine Wandstärke des Behältnisses oder dergleichen. Diese Eigenschaften sind bedeutsam, um hieraus auf eine Qualität des gefertigten Behältnisses zu schließen, beispielsweise darauf, ob die ermittelte Wandstärke innerhalb eines Sollbereichs liegt.

Bei diesen Verfahren werden üblicherweise optische Messeinrichtungen verwendet, beispielsweise wird das Behältnis mit Licht durchleuchtet und eine Transmission bestimmt, aus der sich wiederum auf die Behältniswandstärke rückschließen lässt.

In jüngerer Zeit ist man vermehrt bestrebt, umweltfreundliche Alternativen für Behältnisse herzustellen. Ein Ansatz ist darauf gerichtet, Behältnisse aus Pulpe, insbesondere aus Papier- oder Papppulpe herzustellen. Die ersten Versuche sind vielversprechend. Für derartige Behältnisse tritt jedoch der Nachteil auf, dass diese in der Regel nicht mit sichtbarem Licht inspiziert werden können, weil sie dieses nicht transmittieren.

Im Stand der Technik sind bereits Getränkebehälter in Form von Pappmachebehältern, Pulpebehältern, Fasergussbehältern und dergleichen bekannt, welche üblicherweise in mehreren Prozessschritten aus einer wässrigen Mache hergestellt werden. Nach einer ausreichenden Trocknung kann diese Art von Behältern befüllt und verschlossen werden. Es ist aber weiterhin bekannt, dass Pulpe bei der Trocknung schrumpft. Auf diese Weise kann eine Überprüfung etwa der Maßhaltigkeit erst nach einer ausreichenden Trocknung durchgeführt werden.

Ein weiteres Problem derartiger Behältnisse ist, dass diese oftmals noch eine Restfeuchte aufweisen. Im Stand der Technik ist es möglich, eine Restfeuchtebestimmung bei einer Kartonqualitätskontrolle durchzuführen, beispielsweise über ein Hygrometer. Diese Messung ist dabei berührend mit einem Schwertfühler. Ein Schwertfühler muss mit dem Material in Kontakt sein, bis sich ein ausreichend stabiler Messwert einstellt.

Diese Verfahren eignen sich jedoch nicht für die Untersuchung von den hier relevanten Behältnissen. Insbesondere die berührende Messung würde sich stark auf die Leistung entsprechender Maschinen auswirken.

Wie erwähnt, ist nach der Produktion von derartigen Pulpebehältnissen und damit üblicherweise vor der Weiterverarbeitung (einem Befüllen, Verschließen und dergleichen) noch eine Restfeuchte in der Behältniswandung enthalten. Diese sollte einen definierten Wert nicht überschreiten, damit das Behältnis in der Anlage, bei der Lagerung und dergleichen vernünftig handhabbar ist (insbesondere im Hinblick auf Stabilität, Abriebfestigkeit, Schutz vor Schimmel und dergleichen). Für die Bestimmung der Restfeuchte existieren, wie oben erwähnt, verschiedene etablierte Verfahren oder Ideen wie das Wiegen, eine radiometrische Absorptionsmessung und dergleichen. Diese Verfahren sind jedoch entweder langsam, aufwendig oder teuer oder können nicht exakt unterscheiden zwischen Wandstärkeunterschieden und unterschiedlichen Restfeuchtegehalten.

Der vorliegenden Erfindung liegt daher die Aufgabe zugrunde, ein Bestimmungsverfahren und auch eine Bestimmungsvorrichtung zur Verfügung zu stellen, welche insbesondere für die hier beschriebenen Behältnisse, die aus Pulpe hergestellt sind, angewandt werden können. Diese Aufgaben werden erfindungsgemäß durch die Gegenstände der unabhängigen Patentansprüche gelöst. Vorteilhafte Ausführungsformen und Weiterbildungen sind Gegenstand der Unteransprüche.

Bei einem ersten erfindungsgemäßen Verfahren zum Bestimmen von Behältniseigenschaften wird ein zu untersuchendes Behältnis entlang eines vorgegebenen Transportpfads transportiert und dieses Behältnis weist einen Bodenbereich, einen Grundkörper, der zur Aufnahme des Füllguts dient und einen Mündungsbereich auf. Weiterhin ist dieses Behältnis wenigstens abschnittsweise und bevorzugt im Wesentlichen und besonders bevorzugt vollständig aus einem Papier oder Pappe enthaltenden Material hergestellt und/oder besteht aus einem solchen Material.

Erfindungsgemäß wird wenigstens eine Wandung des Behältnisses zwischen zwei Elektroden angeordnet und an die Elektroden eine elektrische Spannung oder ein elektrisches Wechselfeld angelegt und es wird wenigstens eine elektrische Größe bestimmt, welche für ein elektrisches Feld, welches zwischen diesen Elektroden aufgebaut wird, charakteristisch ist, und aus dieser elektrischen Größe wird wenigstens eine für das Behältnis charakteristische Größe ermittelt.

Bei der hier beschriebenen erfindungsgemäßen Vorgehensweise wird das Behältnis selbst oder eine Wandung oder ein Abschnitt der Wandung untersucht um daraus einen hierfür charakteristischen Wert, beispielsweise eine Restfeuchte oder eine Wandungsdicke abzuleiten. Bevorzugt weist das Material des Behältnisses einen vorbestimmten Flüssigkeit- und insbesondere Wasseranteil auf.

Insbesondere auch durch den besagten Wasseranteil und/oder die Restfeuchte unterscheidet sich das Behältnis von aus dem Stand der Technik bekannten Behältnissen, welche insbesondere aus Glas oder Kunststoff hergestellt ist. Die Anmelderin hat daher Lösungen entwickelt, welche insbesondere zur Anwendung mit wasserenthaltenden Materialien geeignet sind und/oder welche zur Anwendung mit Papier und/oder Pappmaterialien geeignet sind.

Bei einer bevorzugten Ausführungsform besteht das Behältnis aus Papier und/oder Pappe und bevorzugt aus einer papier- oder pappehaltigen Pulpe.

Bevorzugt weist eine Mündung des Behältnisses einen kleineren Querschnitt auf als derjenige des Grundkörpers des Behältnisses.

Daneben wurde auch eine weitere Vorgehensweise der Untersuchung ermittelt, nämlich die oben angegebene Möglichkeit einer Vermessung eines elektrischen Feldes und damit eine kapazitive Messung.

Bei einem bevorzugten Verfahren befindet sich eine der beiden Elektroden während der Bestimmung der elektrischen Größe außerhalb des Behältnisses und die andere Elektrode befindet sich entweder derart außerhalb des Behältnisses, dass das Behältnis (insbesondere wenigstens abschnittsweise) und insbesondere dessen Grundkörper (insbesondere wenigstens abschnittsweise) zwischen den beiden Elektroden angeordnet ist oder die andere Elektrode befindet sich derart abschnittsweise innerhalb des Behältnisses, dass wenigstens ein Wandungsabschnitt des Behältnisses sich zwischen den Elektroden befindet.

Bei einem der beiden hier beschriebenen Verfahren wird daher bevorzugt wenigstens eine Elektrode, nämlich diejenige Elektrode, die sich innerhalb des Behältnisses befindet in der Transportrichtung der Behältnisse mit bewegt.

Bei einem weiteren bevorzugten Verfahren wird zwischen den Elektroden ein elektrisches Wechselfeld und insbesondere ein hochfrequentes elektrisches Wechselfeld aufgebaut. Bevorzugt bildet zumindest wenigstens eine Wandung ein zwischen den Elektroden befindliches Dielektrikum. Bevorzugt befindet sich während der Messung zwischen den Elektroden ausschließlich Luft und wenigstens eine Wandung des Behältnisses.

Eine kapazitive Sensorik ist beispielsweise als Kurzwellenradioelektronik relativ preisgünstig, auch wenn das Handling einer eintauchenden Elektrode in das Behältnis notwendig ist. Allerdings ist es auch möglich, dass bei deutlich höheren Frequenzen bis in den Mikrowellenbereich gemessen werden kann.

Bevorzugt reichen die Messfrequenzen gehen von 2 MHz bis zu 200 GHz, können also im gesamten Radiospektrum von Kurzwelle über UKW, VHF, UHF, Mikrowellen, Radar bis in den Bereich der mm-Wellen legen.

Dabei bieten höhere Frequenzen den Vorteil, dass sie gerichteter messen und dadurch eine Messung auf kleine lokal begrenzte Fläche der Behälterwandung ermöglichen, während niedrigere Frequenzen großflächigere Bereiche erfassen.

Bei einem weiteren bevorzugten Verfahren erfolgt eine oder die Messung bei bzw. mittels mindestens zwei unterschiedlichen (Mess)Frequenzen:
Die erste Messfrequenz zeigt bevorzugt eine stärkere Absorption durch die kohlenwasserstoffhaltigen Holzfasern der Pulpe und die zweite Frequenz zeigt bevorzugt eine stärkere Absorption durch Wasser aus der Restfeuchte in der Wandung.

Dadurch können gleichzeitig die beiden charakteristischen Größen Restfeuchte und Wandstärke bestimmt werden.

Möglicherweise ist auch eine noch wesentlich einfachere Messung nur mit außenliegenden Elektroden möglich. Auf diese Weise könnte eine Restfeuchte nicht nur an einer Stelle auf einer Messfläche, sondern über einen gesamten Umfang des Behältnisses bewertet werden. Durch die Messung mit unten genau beschriebenen übereinander angeordneten Elektroden kann das Behältnis bevorzugt in mehrere vertikale Zonen aufgeteilt werden, die getrennt vermessen werden können.

Bei einem weiteren bevorzugten Verfahren erfolgt die Messung der charakteristischen Größe während einer Bewegung der Behältnisse. Es wird darauf hingewiesen, dass diese bevorzugte Variante für alle die hier vorgeschlagenen Verfahren möglich ist, insbesondere auch für die kapazitive Messung.

Bei einem weiteren bevorzugten Verfahren ist die für das Behältnis charakteristische Größe ein Feuchtewert und insbesondere eine Restfeuchte des Behältnisses und/oder eine Wanddicke wenigstens eines Wandungsabschnitts des Behältnisses.

Bei einem weiteren bevorzugten Verfahren wird das Behältnis während der Messung gedreht und insbesondere bezüglich seiner Längsrichtung gedreht. Auf diese Weise ist eine Vermessung der gesamten Mantelfläche des Behältnisses möglich. Es wird darauf hingewiesen, dass diese bevorzugte Ausführungsform für die hier beschriebene kapazitive Messung geeignet ist.

Bei einem weiteren bevorzugten Verfahren handelt es sich bei der oben erwähnten elektrischen Größe um eine Amplitude und/oder eine Phase eines (insbesondere zwischen den Elektroden aufgebauten) elektrischen Feldes.

Aus dieser Größe und insbesondere aus beiden Größen, d. h. sowohl der Amplitude als auch der Phase des elektrischen Feldes kann eine Restfeuchte gegebenenfalls auch eine Wandungsdicke des Behältnisses ermittelt werden.

Besonders bevorzugt wird aus einer bekannten oder einer konstanten Restfeuchte eine Wanddicke des Behältnisses bestimmt. Hierbei ist zu berücksichtigen, dass in die Berechnung der Restfeuchte auch die Wanddicke eingeht. Ist die Restfeuchte bekannt oder konstant, kann aus den Messungen auch die Wandstärke ermittelt werden.

Bei einem bevorzugten Verfahren wird eine Elektrode in das Behältnis geführt bzw. eingetaucht. Dies erfolgt bevorzugt über die Mündungsöffnung des Behältnisses.

Bevorzugt sind ein oder mehrere Elektroden außerhalb des Behältnisses angeordnet. Bevorzugt erfolgt, wie gesagt, eine Messung der Amplitude und Phase besonders bevorzugt bei unterschiedlichen Hochfrequenzen. Besonders bevorzugt werden auch unterschiedliche Geometrien (insbesondere der Elektroden) verwendet, um eine gleichzeitige Feuchte- und Wandstärkenvermessung vorzunehmen.

Bei einer bevorzugt durchgeführten Drehung des Behältnisses (beispielsweise durch Abrollen) ist eine Messung der gesamten Mantelfläche möglich.

In dem oben erwähnten Fall einer konstanten oder zumindest bekannten Restfeuchte ist eine Kompensation und damit eine direkte Wandstärkenberechnung möglich. Daneben ist auch eine Messung mit außenliegenden Elektroden möglich, was eine Messung an einem Transporteur im freien Durchlauf ermöglicht.

Bei einem weiteren bevorzugten Verfahren erfolgt die Bestimmung der genannten Eigenschaften und/oder die Messung berührungslos. Die genannten Verfahren haben den Vorteil, dass sie nicht zu einer Begrenzung der Produktionsleistung führen.

Das genannte Verfahren erlaubt eine Qualitätskontrolle des produzierten Behältnisses. Diese gemessenen Werte können auch verwendet werden, um eine anschließende Trocknung der Behältnisse durchzuführen. Auf diese Weise kann auch eine Closed-Loop-Regelung beispielsweise des Materialeinsatzes, eines Materialmischungsverhältnisses, eines Wasseranteils, einer Trocknungsenergie, einer Trocknungszeit und dergleichen vorgenommen werden.

Bevorzugt werden die hier beschriebenen Messungen an einem unbefüllten Behältnis durchgeführt. Bevorzugt werden die hier beschriebenen Messungen an einem unverschlossenen Behältnis durchgeführt.

Bevorzugt werden die hier beschriebenen Messungen an einem erstmalig zu befüllenden Behältnis durchgeführt.

Die vorliegende Erfindung ist weiterhin auf eine Vorrichtung zum Bestimmen von Behältniseigenschaften gerichtet. Diese weist eine Transporteinrichtung auf, welche ein zu untersuchendes Behältnis (und bevorzugt eine Vielzahl von zu untersuchenden Behältnissen) entlang eines vorgegebenen Transportpfads transportiert, wobei dieses Behältnis einen Bodenbereich, einen Grundkörper, der zur Aufnahme eines Füllguts dient und einen Mündungsbereich aufweist und wobei dieses Behältnis wenigstens abschnittsweise und bevorzugt vollständig aus einem Papier oder Pappe enthaltenen Material besteht.

Erfindungsgemäß weist die Vorrichtung wenigstens zwei Elektroden auf, welche derart angeordnet sind, dass wenigstens eine Wandung des Behältnisses zeitweise zwischen diesen zwei Elektroden angeordnet ist und wobei an die Elektroden eine elektrische Spannung anlegbar ist und weiterhin eine Messeinrichtung vorgesehen ist, welche eine elektrische Größe bestimmt, welche für ein elektrisches Feld, welches zwischen den Elektroden aufbaubar ist und/oder aufgebaut wird, charakteristisch ist. Weiterhin ist eine Auswerteeinrichtung vorgesehen, welche aus dieser elektrischen Größe wenigstens eine für das Behältnis charakteristische Größe ermittelt.

Es wird daher auch vorrichtungsseitig vorgeschlagen, dass eine Messung des Behältnisses berührungslos erfolgt. Bevorzugt erfolgt die Messung auch Inline, d. h. während eines Herstellungsbetriebs.

Zur Messung der jeweiligen Größen werden wie oben erwähnt, kapazitive Messungen eingesetzt.

Bevorzugt handelt sich bei der für das Behältnis charakteristischen Größe um eine Feuchte und/oder einen Feuchtegehalt und/oder eine Wanddicke, insbesondere eine Wanddicke in einem Bereich des Bodens oder des Grundkörpers des Behältnisses.

Besonders bevorzugt, handelt es sich bei der Transporteinrichtung um ein (insbesondere einbahniges) Transportband oder eine Transportkette. Besonders bevorzugt erfolgt der Transport der Behältnisse entlang einer geradlinigen Richtung und insbesondere in einem stehenden Zustand.

Es wäre jedoch auch möglich, dass die Transporteinrichtung einen drehbaren Träger aufweist, an dem eine Vielzahl von Halteelementen zum Halten der Behältnisse angeordnet ist oder der Behälter im Neckhandling transportiert wird insbesondere in oder aus einer Kavität.

Die oben beschriebenen Elektroden erstrecken sich bevorzugt in einer Längsrichtung des oder der Behältnisse, wobei die Behältnislängsrichtung bevorzugt von der Mündung zu dem Boden der Behältnisse reicht.

Bei einer bevorzugten Ausführungsform weist wenigstens eine Elektrode und insbesondere eine außenliegende Elektrode eine Vielzahl von separat ansteuerbaren oder mit unterschiedlicher Frequenz beaufschlagbaren Elektrodenabschnitten auf, wodurch bevorzugt zwischen jedem einzelnen Elektrodenabschnitt dieser Elektrode und der anderen Elektrode ein elektrisches Feld aufbaubar ist. Auf diese Weise ist beispielsweise eine Restfeuchtemessung in Abhängigkeit von einer Höhenposition des Behältnisses möglich.

Bei einer weiteren bevorzugten Ausführungsform weist die Vorrichtung eine Dreheinrichtung auf, welche dazu geeignet und bestimmt ist, die Behältnisse insbesondere bezüglich ihrer Längsrichtung zu drehen. Es wird darauf hingewiesen, dass diese Ausgestaltung sowohl für die Messungen mittels elektromagnetischer Strahlung als auch für die kapazitive Messungen anwendbar ist.

Dabei es ist in einer Variante möglich, dass diese Drehung erfolgt, wenn die Messung durchgeführt wird, es wäre jedoch auch möglich, dass mehrfach abschnittsweise entlang des Transportpfads gedreht und auch gemessen wird.

Bevorzugt kann die Dreheinrichtung beispielsweise Seitenführungsbänder aufweisen, welche an den Grundkörper der Behältnisse anlegbar sind und so auch dessen Drehung bewirken.

Die vorliegende Erfindung ist weiterhin auf eine Anordnung zum Behandeln von Behältnissen mit einer Vorrichtung der oben beschriebenen Art gerichtet sowie einer Trocknungseinrichtung zum Trocknen der Behältnisse, wobei bevorzugt diese Trocknungseinrichtung in Abhängigkeit von der für das Behältnis charakteristischen Größe, insbesondere einer Restfeuchte steuerbar ist. Die Trocknung erfolgt bevorzugt mit eingeblasener Luft, welche die Trocknungskammer mit dem darin befindlichen Behälter durchströmt. Die zu steuernden Parameter können z.B. die Temperatur, die Strömungsgeschwindigkeit oder die Höhe des Unterdrucks der Trocknungsluft sein.

Bevorzugt ist diese Trocknungseinrichtung nach der oben beschriebenen Vorrichtung angeordnet. Besonders bevorzugt erlaubt die Steuerungseinrichtung eine Regelung und insbesondere eine Regelung in Form eines Closed-Loops bzw. eines geschlossenen Regelkreises.

Weiterhin kann die Anordnung mehrere der oben beschriebenen Vorrichtungen aufweisen. So ist es möglich, dass beispielsweise hintereinander entlang des Transportpfades unterschiedliche Messungen durchgeführt werden, beispielsweise eine Messung mittels nahinfrarotem Licht sowie eine kapazitive Messung.

Die Messergebnisse können dabei beispielsweise redundant verwendet werden, es wäre doch auch möglich, dass mit einer ersten Messung eine Restfeuchte und mit einer weiteren Messung eine Wanddickenbestimmung vorgenommen wird. Der Fachmann erkennt, dass die hier vorgeschlagenen Vorrichtungen miteinander kombiniert werden können, beispielsweise eine Messung mit Röntgenstrahlung mit einer Messung mit infrarotem Licht oder auch eine kapazitive Messung mit einer Wärmemessung oder weitere Ausgestaltungen.

Weitere Vorteile und Ausführungsformen ergeben sich aus den beigefügten Zeichnungen:
Darin zeigen:
- Fig. 1: eine schematische Darstellung einer Vorrichtung;
- Fig. 2: eine erste Darstellung einer kapazitiven Messung; und
- Fig. 3: eine weitere Darstellung einer kapazitiven Messung.

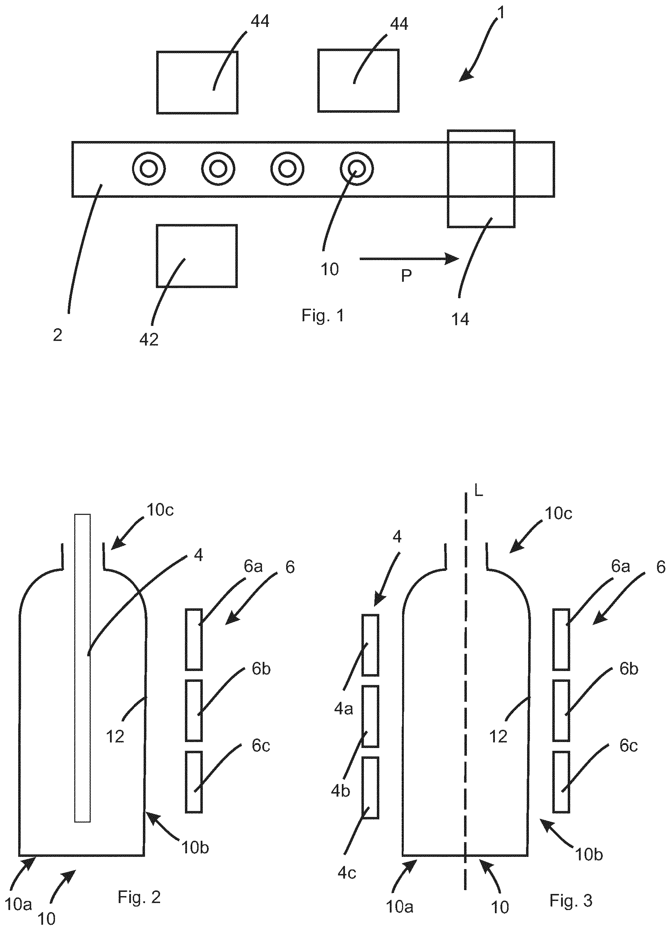

Fig. 1 zeigt eine grobschematische Darstellung einer Vorrichtung 1. Dabei ist eine Transporteinrichtung 2, wie etwa ein Transportband vorgesehen, welches Behältnisse 10 entlang eines Transportpfads P transportiert. Diese Figur zeigt zwar eine Vorrichtung, welche für Messungen mit elektromagnetischer Strahlung ausgelegt ist, die Verwendung des gezeigten Transportbandes kommt jedoch auch für ein kapazitive Messung in Betracht Diese Vorrichtung und insbesondere ist in entsprechender Weise auch für die hier beschriebene kapazitive Messung verwendbar.

Das Bezugszeichen 42 kennzeichnet eine Strahlungseinrichtung, welche Strahlung auf die Behältnisse 10 einstrahlt und das Bezugszeichen 44 eine Sensoreinrichtung, welche die von den Behältnissen transmittierte oder reflektierte Strahlung aufnimmt. Im Falle der Erfindung könnten an den beiden Seiten des Transportbands Elektroden vorgesehen sein, welche die im Rahmen der vorliegenden Anmeldung beschriebenen kapazitiven Messungen durchführen.

Auch wäre es möglich, dass eine erste Elektrode neben dem Transportpfad angeordnet ist und eine weitere Elektrode in die Behältnisse eingeführt wird und/oder sich mit den Behältnissen mitbewegt.

Das Bezugszeichen 14 kennzeichnet eine Trocknungseinrichtung, welche zum Trocknen der Behältnisse 10 dient. Bevorzugt ist eine Steuerungseinrichtung zum Steuern dieser Trockungseinrichtung vorgesehen, welche diesen Trocknungsvorgang insbesondere in Abhängigkeit von einem von der Sensoreinrichtung festgestellten Messwert wie insbesondere einer ermittelten Restfeuchte des Behältnisses steuert.

Wie oben erwähnt, handelt es sich bei den Behältnissen um Behältnisse, welche aus Papier oder Pappe und insbesondere einer aus Papier oder Pappe gebildeten Pulpe aufgebaut ist.

Die Fig. 2 und 3 zeigen Anordnungen für eine kapazitive Messung. Bei der in Fig. 2 gezeigten Variante wird eine Elektrode 4 in das Behältnis eingeführt und es wird das elektrische Feld zu einer zweiten Elektrode 6, die hier drei Einzelelektroden 6a, 6b und 6c aufweist, bestimmt. Aus den Charakteristika dieses elektrischen Feldes insbesondere der Amplitude und der Phase kann auf die Restfeuchte in der Wandung 10b des Behältnisses geschlossen werden.

Fig. 3 zeigt eine weitere Variante, wobei hier zwei Elektroden 4 (4a, 4b, 4c) und 6 (6a, 6b, 6c) jeweils außenseitig angeordnet sind. Der Vorteil dieser Messung besteht darin, dass keine Elektrode mit dem Behältnis, welches sich auch hier senkrecht zur Figurenebene bewegt, mitbewegt werden muss.

Die Anmelderin behält sich, vor sämtliche in den Anmeldungsunterlagen offenbarten Merkmale als erfindungswesentlich zu beanspruchen, sofern sie einzeln oder in Kombination gegenüber dem Stand der Technik neu sind. Es wird weiterhin darauf hingewiesen, dass in den einzelnen Figuren auch Merkmale beschrieben wurden, welche für sich genommen vorteilhaft sein können. Der Fachmann erkennt unmittelbar, dass ein bestimmtes in einer Figur beschriebenes Merkmal auch ohne die Übernahme weiterer Merkmale aus dieser Figur vorteilhaft sein kann. Ferner erkennt der Fachmann, dass sich auch Vorteile durch eine Kombination mehrerer in einzelnen oder in unterschiedlichen Figuren gezeigter Merkmale ergeben können.

## Patentansprüche

1. Verfahren zum Bestimmen von Behältniseigenschaften, wobei ein zu untersuchendes Behältnis (10) entlang eines vorgegebenen Transportpfads transportiert wird und dieses Behältnis (10) einen Bodenbereich (10a), einen Grundkörper (10b) der zu Aufnahme eines Füllguts dient und einen Mündungsbereich aufweist und wobei dieses Behältnis (10) wenigstens abschnittsweise aus einem Papier- oder Pappe enthaltenden Material besteht,
**dadurch gekennzeichnet, dass**
wenigstens eine Wandung (12) des Behältnisses (10) zwischen zwei Elektroden (4, 6) angeordnet wird und an die Elektroden (4, 6) eine elektrische Spannung angelegt wird und wenigstens eine elektrische Größe bestimmt wird, welche für ein elektrisches Feld, welches zwischen Elektroden aufgebaut wird, charakteristisch ist und aus dieser elektrischen Größe wenigstens eine für das Behältnis (10) charakteristische Größe ermittelt wird.

2. Verfahren nach Anspruch 1,
**dadurch gekennzeichnet**, das
eine der beiden Elektroden (6) sich während der Bestimmung der elektrischen Größe außerhalb des Behältnisses (10) befindet und die andere Elektrode (4) sich entweder derart außerhalb des Behältnisses (10) befindet, dass das Behältnis (10) zwischen den beiden Elektroden (4, 6) angeordnet ist oder die andere Elektrode (4) sich wenigstens derart abschnittsweise innerhalb des Behältnisses (10) befindet, dass wenigstens ein Wandungsabschnitt des Behältnis (10) sich zwischen den Elektroden (4, 6) befindet.

3. Verfahren nach wenigstens einem der vorangegangenen Ansprüche,
**dadurch gekennzeichnet, dass**
das Behältnis (10) aus einer papier- oder pappehaltigen Pulpe besteht.

4. Verfahren nach wenigstens einem der vorangegangenen Ansprüche,
**dadurch gekennzeichnet, dass**
zwischen den Elektroden (4, 6) ein elektrisches Wechselfeld und insbesondere ein hochfrequentes elektrisches Wechselfeld aufgebaut wird.

5. Verfahren nach wenigstens einem der vorangegangenen Ansprüche,
**dadurch gekennzeichnet, dass**
die Messung bei mindestens zwei unterschiedlichen Frequenzen erfolgt.

6. Verfahren nach wenigstens einem der vorangegangenen Ansprüche,
**dadurch gekennzeichnet, dass**
die Messung der charakteristischen Größe während einer Bewegung der Behältnisse (10) erfolgt.

7. Verfahren nach wenigstens einem der vorangegangenen Ansprüche,
**dadurch gekennzeichnet, dass**
die für das Behältnis (10) charakteristische Größe ein Feuchtewert und insbesondere Restfeuchte des Behältnisses und/oder eine Wanddicke wenigstens eines Wandungsabschnitts des Behältnisses (10) ist.

8. Verfahren nach wenigstens einem der vorangegangenen Ansprüche,
**dadurch gekennzeichnet, dass**
die elektrische Größe eine Amplitude und/oder eine Phase des elektrischen Feldes ist.

9. Verfahren nach wenigstens einem der vorangegangenen Ansprüche,
**dadurch gekennzeichnet, dass**
bei bekannter und oder konstanter Restfeuchte eine Wanddicke bestimmt wird.

10. Verfahren nach wenigstens einem der vorangegangenen Ansprüche,
**dadurch gekennzeichnet, dass**
die wenigstens eine für das Behältnis (10) charakteristische Größe in der Längsrichtung (L) des Behältnisses abschnittsweise ermittelt wird und/oder die wenigstens eine für das Behältnis (10) charakteristische Größe in Abhängigkeit von der Längsrichtung (L) des Behältnisses (10) bestimmt wird.

11. Verfahren nach wenigstens einem der vorangegangenen Ansprüche,
**dadurch gekennzeichnet, dass**
das Behältnis während der Messung gedreht und insbesondere bezüglich seiner Längsrichtung gedreht wird.

12. Vorrichtung zum Bestimmen von Behältniseigenschaften, mit einer Transporteinrichtung (2) welche ein zu untersuchendes Behältnis (10) entlang eines vorgegebenen Transportpfads transportiert wobei dieses Behältnis (10) einen Bodenbereich (10a), einen Grundkörper (10b) der zu Aufnahme eines Füllguts dient und einen Mündungsbereich (10c) aufweist und wobei dieses Behältnis wenigstens abschnittsweise und bevorzugt vollständig aus einem Papier- oder Pappe enthaltenden Material besteht,
**dadurch gekennzeichnet, dass**
die Vorrichtung wenigstens zwei Elektroden (4, 6) aufweist, welche derart angeordnet sind, dass wenigstens eine Wandung (12) des Behältnisses (10) zeitweise zwischen diesen zwei Elektroden (4, 6) angeordnet ist wobei an die Elektroden eine elektrische Spannung anlegbar ist und eine Messeinrichtung vorgesehen ist, welche wenigstens eine elektrische Größe bestimmt, welche für ein elektrisches Feld, welches zwischen den Elektroden aufgebaut wird, charakteristisch ist und weiterhin eine Auswerteeinrichtung vorgesehen ist welche aus dieser elektrischen Größe wenigstens eine für das Behältnis (10) charakteristische Größe ermittelt.

13. Vorrichtung nach dem vorangegangenen Anspruch,
**dadurch gekennzeichnet, dass**
wenigstens eine Elektrode (6) eine Vielzahl von separat ansteuerbaren Elektrodenabschnitten (6a, 6b, 6c) aufweist, wodurch bevorzugt zwischen jedem einzelnen Elektrodenabschnitt dieser Elektrode und der anderen Elektrode ein elektrisches Feld aufbaubar ist.

14. Vorrichtung (1) nach wenigstens einem der vorangegangenen Ansprüche,
**dadurch gekennzeichnet, dass**
die Vorrichtung (1) eine Dreheinrichtung aufweist, welche dazu geeignet und bestimmt ist, die Behältnisse bezüglich ihrer Längsrichtung zu drehen.

15. Anordnung zum Behandeln von Behältnissen mit einer Vorrichtung (1) nach wenigstens einem der vorangegangenen Ansprüche und mit einer Trocknungseinrichtung (12) zum Trocknen der Behältnisse, wobei die Trocknungseinrichtung in Abhängigkeit von der für das Behältnis charakteristischen Größe steuerbar ist.
